Europäisches Patentamt

**European Patent Office**

Office européen des brevets

(11) Publication number:

**0 313 689**
**A1**

# EUROPEAN PATENT APPLICATION

(21) Application number: **87309448.6**

(22) Date of filing: **26.10.87**

(51) Int. Cl.⁴: **A45C 7/00 , A45C 11/00**

(43) Date of publication of application:
**03.05.89 Bulletin 89/18**

(84) Designated Contracting States:
**AT BE CH DE ES FR GB GR IT LI LU NL SE**

(71) Applicant: **A.L. MOTHER'S HELPERS
INDUSTRIES INC.
5518 Silverson Avenue
Montreal Quebec H4V 2G5(CA)**

(72) Inventor: **Thomas, Allan
5518 Silverson Avenue
Montreal Quebec H4V 2G5(CA)**
Inventor: **Finkel, Henry
342 Elm Avenue
Westmount Quebec H3Z 1Z5(CA)**

(74) Representative: **Spence, Anne et al
Spence & Townsend Mill House Wandle
Road
Beddington Croydon Surrey CR0 4SD(GB)**

(54) **Disposable kit for toiletry or like products.**

(57) A single use, disposable kit (10) receives and retains, toiletries (74 to 78) and a diaper (80) for the care of a baby in a sanitary, tamper proof fashion. The kit is made from thin plastics film (16, 18) that is folded over upon itself to provide a pocket (30) and a pouch (40) and a compartment or compartments for the products. The pouch (40) is then folded over and tucked into the pocket (30) and sealed thereto with a label (12). The products are enveloped within the interior of the kit and are protected against degradation. After using, the soiled contents of the kit can be reinserted and enveloped within the body of the kit for disposition.

*FIG. 3.*

## DISPOSABLE KIT FOR TOILETRY OR LIKE PRODUCTS

The present invention pertains generally to kits suitable for retaining sundry toiletry products or first aid supplies. More specifically, the present invention relates to an inexpensive, disposable kit that retains toiletries, preferably for the care of a baby, in a sanitary, tamper-proof fashion.

An inexpensive, disposable kit that could be manufactured, and marketed, at a reasonable price has significant commercial potential. Such a kit would find a ready audience among parents, babysitters, nurseries, hospitals and other segments of the population. A disposable kit, in essence, should appeal to the same persons as presently use disposable diapers, disposable bibs, and other disposable items of this ilk. Furthermore, if the kit were produced in such a manner that the products included therein were maintained in a sanitary condition until usage, the chances of transmitting disease would be considerably reduced, a major consideration for schools, hospitals, day care centres, and the like. Lastly, if the kit were sealed in a tamper-indicating manner, the user would be guaranteed, for all practical purposes, that the contents of the kit had not been adulterated, tainted, or otherwise rendered unsuitable for their intended purposes.

Furthermore, after the seal on the package for the kit has been broken, and the contents of the kit used in the intended fashion, the same package could be re-used to receive, and retain, the used disposable diaper and other used baby care products. The configuration of the package could permit the package to be closed, once again, thus avoiding further pollution to the surroundings, the person handling the baby and the baby itself. Also the disposition of the soiled material is simplified under improved hygenic conditions.

While U.S. Specifications 2533850, 1606115 and 4221221 disclose packages for toiletries none of these provided an inexpensive, disposable, single use kit for retaining the numerous products necessary to change and cleanse, a baby's bottom, and/or to provide a readily available hygienically safe package for disposing of the soiled diaper and baby care products used in the cleaning operation.

An object of the invention is to provide an improved disposable kit for toiletry or first aid products particularly for the care of a baby.

Accordingly in its broadest aspect the present invention provides an inexpensive, disposable kit that retains therewithin in a sanitary manner adequate supplies of toiletries and which is such that soiled materials may be returned to a pouch defined within the kit for ready disposal. Preferably the pouch contains a babies diaper and the pouch and contents folds over and inserts into a pocket and is sealed thereto.

In a preferred form the invention provides a sanitary disposable kit comprising a body formed from a sheet of material having a leading section and a trailing section situated at opposite ends of the body, the leading section being folded about itself and being secured to the upper surface of the body to form a pocket the trailing section being folded about itself and being secured to the upper surface of the body to form a pouch, the pouch being longer than the pocket, the free edge of the pocket being spaced a short distance from the free edge of the pouch to permit access to the pouch when the body is in its open position, at least one panel secured to the trailing section to define at least one compartment on the upper surface of the trailing section, toiletry or first aid products stored within the compartment and/or pouch, the trailing section being inserted in the pocket after the trailing section has been folded about itself, and sealing means securing the bottom surface of the trailing section to the upper surface of the leading section so that products are enveloped and sealed in a sanitary manner within the confines of the kit prior to using the kit, and in which soiled products can be re-inserted into the pouch prior to refolding the kit and disposing of same after use. Preferably the body is made of thin plastics material film.

Embodiments of kit, in accordance with this invention, will now be described, by way of example only, with reference to the accompanying drawings of which:-

Fig. 1 is a perspective view of a disposable, baby change kit shown in its sealed condition;

Fig. 2 is a top plan view of the components of the kit prior to assembling same;

Fig. 3 is a vertical cross-sectional view of the kit, such view being taken along line 3-3 in Fig. 2 and in the direction indicated;

Fig. 4 is an exploded perspective view of the kit, in its opened condition; and

Fig. 5 is a perspective view of another embodiment of kit for men's toiletries

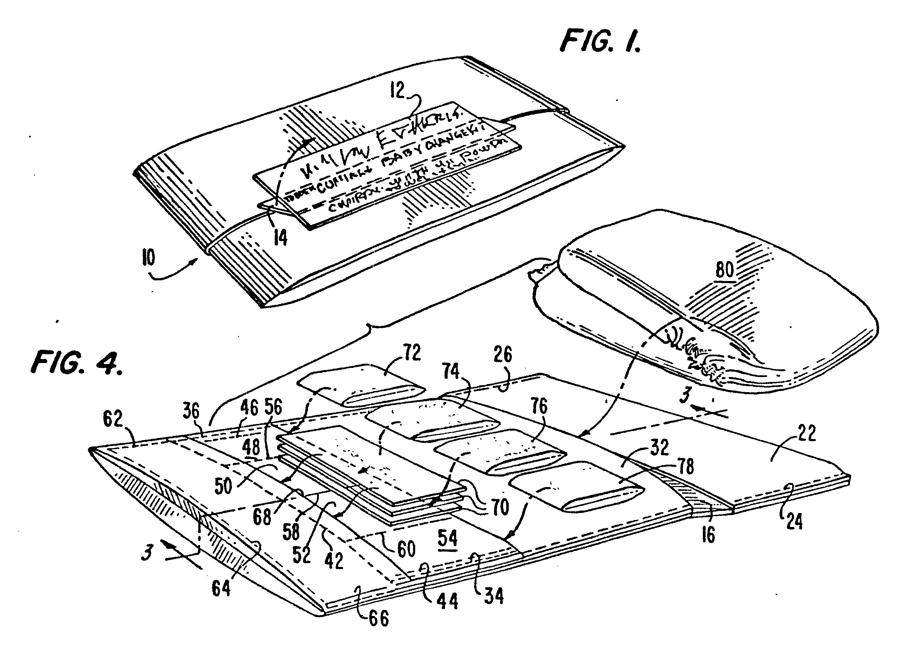

Figs. 1 to 4 depict a kit 10 in the form of a baby change kit.

A pressure-sensitive label 12 extends partially across the front of the kit 10 and retains the kit in its sealed and sanitary, condition. The lable recites the various products contained within the kit; for example, in the case of a baby change kit, the label notes that the kit includes a diaper, a packet of baby powder, a packet of petroleum jelly, a pre-moistened towel, a dry towel, soap and the like.

A tear strip 14 may be incorporated into the label, and the user gains access to the contents of the kit by pulling upwardly on the tear strip, thus severing the label and permitting the kit to be opened. Once the tear strip has been utilized, the label clearly indicates that someone has attempted to gain access to the contents of the kit, and that the sanitary condition of the kit may have been compromised.

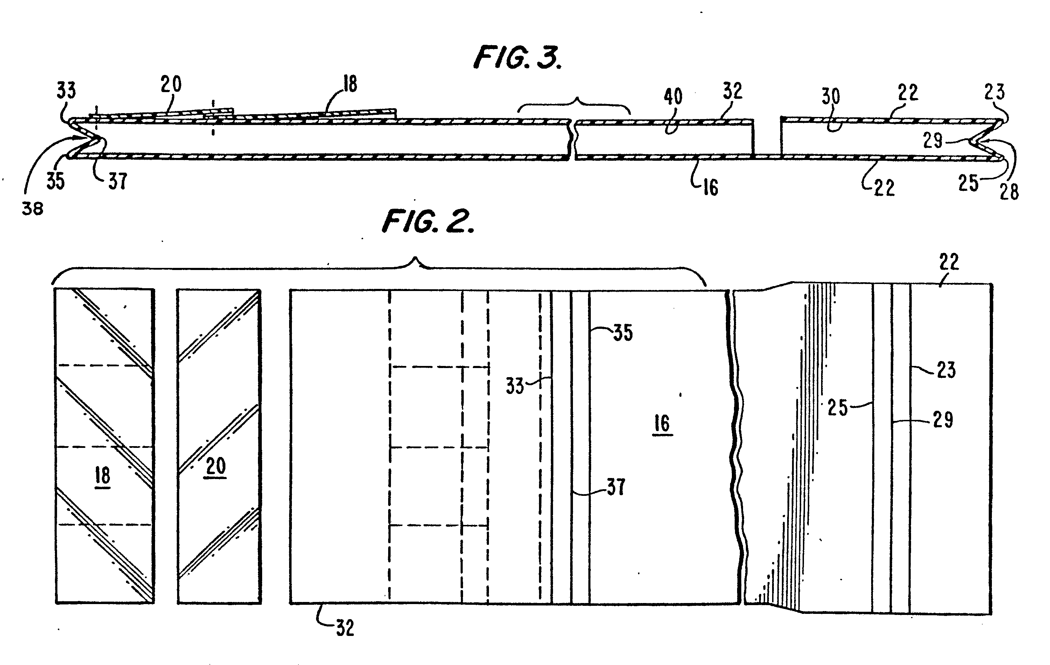

While Fig. 1 shows the kit in its folded, and sealed condition, Fig. 2 illustrates that the kit 10 comprises three components of thin plastic film; the components are identified as body 16, first panel 18, and second panel 20. The blank from which body 16 is formed is generally rectangular in shape, but has a leading section 22 that is slightly greater in width that the remainder of the body. The top and bottom surfaces of the body are both finished and decorated.

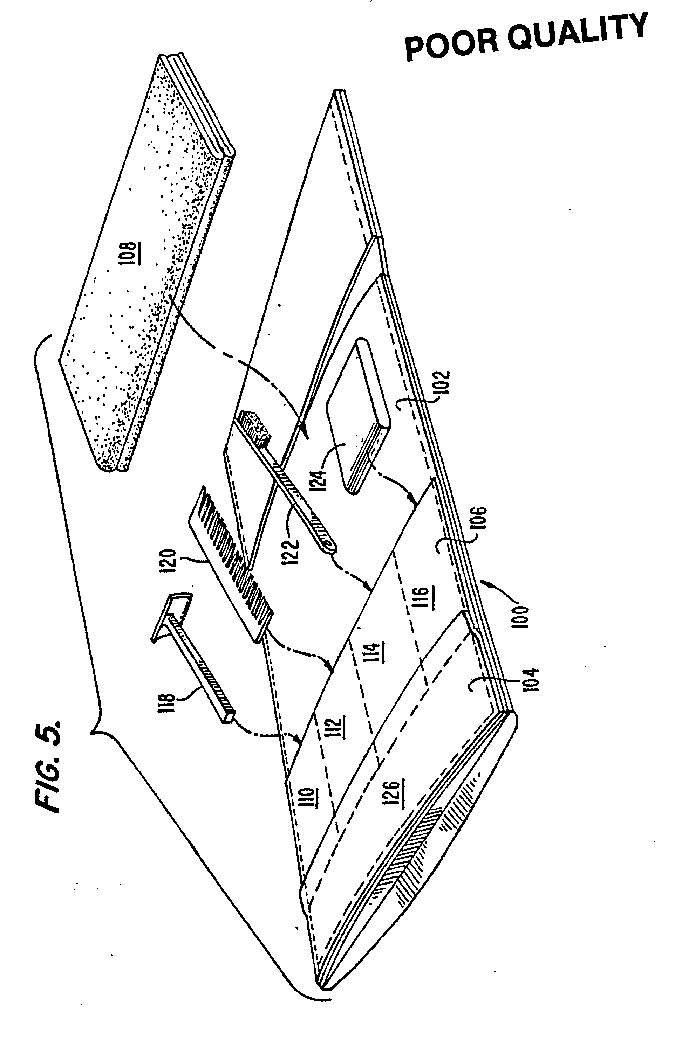

To form the kit 10 from components 16, 18 and 20, the leading section 22 of body 16 is folded about line 23, and then about line 25. The leading section 22 is then heat-sealed to body 16 along longitudinal lines 24 and 26 (visible in Fig. 4). A bellows fold 28 is formed at one end of the body by flexing same inwardly, along line 29, and the portion between folds 24, 26 form a pocket base which contributes to the depth of the pocket 30 defined between the folded over portions of leading section 22 and the upper surface of the body 16, as shown in Fig. 3.

The trailing section 32 of the blank that is formed into body 16 is also folded about itself along fold lines 33 and 35 until the free edge of section 32 is located a short distance from pocket 30. The trailing section 32 is then heat-sealed to the underlying portion of blank 16 along lines 34, 36. A bellows fold 38 is formed at the end of the body and the portion between folds 33,35 forms a base which contributes to the depth of pouch 40 defined between folded over portions of trailing section 32, as shown in Fig. 3.

The first panel 18 may be formed of a rectangle of clear vinyl plastic, while the body 16 is formed of a finished, and visually pleasing, decorated polyethylene film. The panel 18 is approximately equal in width to body 16. The first panel 18 is heat-sealed to body 16 along transverse line 42, and is also heat-sealed to body 16 along lines 44 and 46. The panel 18 is subdivided into a plurality of small compartments 48,50,52 and 54 by securing the first panel to the underlying body 16 along spaced lines 56,58 and 60. If desired, the body 16 may be scored or otherwise marked so that the space lines at which heat sealing or welding takes place are properly oriented.

The second panel 20, which is also formed of a rectangle of clear vinyl plastic and is equal in width to body blank 16, is then secured to the body 16 along lines 62, 64 and 66. The flap 20 overlaps, to a limited extent, flap 18. A large compartment 68 is formed between the flap 20 and the upper surface of body blank 16, as shown in Fig. 3.

Fig. 4 depicts the manner in which the kit is filled with products for the care of a baby. A coupon 70 is slipped into compartment 68, while compartments 48, 50, 52 and 54 are filled with a dry towel packet 72, a packet of baby powder 74, a wet towel packet 76, and a packet of soap 78. A disposable, paper diaper 80 is inserted into the large pouch 40 defined by the folded over trailing section 32 of the body 16.

After the various baby care products are inserted into the various compartments, and into the pouch 40, the trailing section 32 is folded over and slipped into the pocket 30 in the slightly wider leading section 22. The added width of the head facilitates the insertion step. The pressure-sensitive label 12 is applied. Since the foot section fits neatly within the pocket 30, the kit, in effect, has been folded over itself and the contents of the kit are securely retained within the interior of the kit in a sanitary fashion. Furthermore, because of the relatively simple configuration of the kit, the kit can be used once, and then discarded.

The pouch 40 is sized to receive a disposable diaper 80, which, after use, can be re-inserted thereinto. The kit can then be refolded and the pouch end inserted in the pocket and subsequently disposed of in a relatively pollution free manner with a minimum amount of handling. A means for resealing, which indicated that the kit had been used, could be provided.

Fig. 5 shows an alternative embodiment of kit. More specifically, the alternative kit 100 comprises a body 102, a first panel 104, and a second panel 106 joined together in the same fashion as kit 10. However, a disposable towel 108, of linen or paper, is inserted into the pouch. The pockets 110, 112, 114 and 116 are defined in the first flap 104 by appropriate weld lines, are filled with a disposable razor 118, a comb 120, a toothbrush 122, and a bar of soap 124. Coupons or similar items (not shown) may be inserted into the larger pocket 126. After the body has been filled with the toiletries needed for the kit, the trailing section 128 of the body is folded over and inserted into the slightly larger head 130; a label (not shown) is then applied to the kit to seal same.

The kit may be filled with different items and used for numerous other purposes. For example, the kit could be filled with sample items for a trade show, or might contain medical supplies to serve as an emergency first-aid kit.

Also, while the body of the kit is preferably formed from a .003 inch (.0076 cms) thick sheet of polyethylene, properly slit, folded and heat-sealed, the same principles may be applied to kits formed of other plastics or executed in cloth.

## Claims

1. A sanitary disposable kit (10) comprising a body (16) formed from a sheet of material having a leading section (22) and a trailing section (32) situated at opposite ends of the body, the leading section being folded about itself and being secured to the upper surface of the body to form a pocket (30), the trailing section being folded about itself and being secured to the upper surface of the body to form a pouch (40), the pouch being longer than the pocket, the free edge of the pocket being spaced a short distance from the free edge of the pouch to permit access to the pouch when the body is in its open position, at least one panel (18,20) secured to the trailing section to define at least one compartment (48,50,52,54,68) on the upper surface of the trailing section, toiletry or first aid products (72, 74, 76, 78, 80) stored within the compartment and/or pouch, the trailing section (32) being inserted in the pocket (30) after the trailing section has been folded about itself, and sealing means securing the bottom surface of the trailing section to the upper surface of the leading section so that products are enveloped and sealed in a sanitary manner within the confines of the kit prior to using the kit, and in which soiled products can be re-inserted into the pouch prior to refolding the kit and disposing of same after use.

2. A kit according to Claim 1 in which the pocket (30) has a greater width than the pouch (40).

3. A kit according to Claim 1 or Claim 2 in which the pocket (30) and the pouch (40) each have a base formed by a plurality of folds in the body.

4. A kit according to Claim 3 in which the pocket and pouch each have a base formed by a bellows fold and the bases are of equal height so that the pocket and pouch are substantially equal in height.

5. A kit according to any of Claims 1 to 4 in which the sealing means includes a pressure sensitive label (12) and tear strip (14) extending across the label, the removal of the tear strip serving as a visual indicator that the contents of the kit has been accessed.

6. A kit according to any of Claims 1 to 5 in which the body is formed from plastics sheet material.

7. A kit according to any of Claims 1 to 6 containing babycare products including a diaper.

FIG. I.

FIG. 4.

# FIG. 3.

33     20        18              40   32      30    22    23

35    37            16        22    25   28

38

# FIG. 2.

18     20             35           22

33          23

16         25     29

37

32

EP 0 313 689 A1

FIG. 5.

118
120
108
110
112
122
114
124
126
116
102
106
104
100

EP 0 313 689 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,A | US-A-1 606 115 (WILLIAMS)<br>--- | | A 45 C 7/00<br>A 45 C 11/00 |
| A | US-A-4 391 370 (DALBO)<br>--- | | |
| A | US-A-2 710 638 (FORD)<br>--- | | |
| A | GB-A-1 024 111 (DICKINSON)<br>--- | | |
| A | US-A-3 120 297 (RILEY)<br>--- | | |
| A | US-A-1 545 730 (BENSON)<br>----- | | |

TECHNICAL FIELDS
SEARCHED (Int. Cl.4)

A 45 C
B 65 D
A 61 F

The present search report has been drawn up for all claims

| Place of search | Date of completion of the search | Examiner |
|---|---|---|
| THE HAGUE | 27-06-1988 | SIGWALT C. |